# EUROPEAN PATENT SPECIFICATION

(11) **EP 4 414 705 B1**
(45) Date of publication and mention of the grant of the patent: **02.09.2026**
(21) Application number: 22911634.8
(22) Date of filing: 17.11.2022
(51) Int. Cl.: G01N 33/543, G01N 21/84, G01N 21/17

(54) **METHOD FOR CONDUCTING QUANTITATIVE ANALYSIS IN RAPID DIAGNOSTIC TEST**
VERFAHREN ZUR DURCHFÜHRUNG EINER QUANTITATIVEN ANALYSE BEI EINEM SCHNELLEN DIAGNOSTISCHEN TEST
PROCÉDÉ POUR EFFECTUER UNE ANALYSE QUANTITATIVE DANS UN TEST DE DIAGNOSTIC RAPIDE

(30) Priority: 21.12.2021 KR 20210183939
(43) Date of publication of application: 14.08.2024
(73) Proprietor: Boditech Med Inc., Chuncheon-si, Gangwon-do 24398 (KR)
(72) Inventor: KIM, Byeong Chul, Chuncheon-si, Gangwon-do 24304 (KR); BYUN, Hee Jung, Chuncheon-si, Gangwon-do 24209 (KR); HWANG, Min Woong, Chuncheon-si, Gangwon-do 24221 (KR)
(74) Representative: Louis Pöhlau Lohrentz
(86) International application number: PCT/KR2022/018226
(87) International publication number: WO 2023/120997

(56) References cited:
- WO-A1-2019/215199
- GB-A- 2 594 939
- JP-A- 2016 206 060
- KR-A- 20160 052 442
- KR-A- 20210 085 411
- KR-B1- 102 175 653
- US-A1- 2014 240 491
- US-A1- 2019 285 551
- US-A1- 2020 300 697
- US-A1- 2021 089 814

## Description

### FIELD OF THE INVENTION

The present invention relates to a quantitative analysis method in rapid diagnostic tests, more specifically, to a method for performing quantitative analysis in a rapid diagnostic test by using a lateral flow cartridge containing a mark identifiable by visible light and a mobile communication terminal equipped with a camera.

### BACKGROUND ART

In a rapid diagnostic test, which is one of point-of-care testings, a lateral flow cartridge using gold nanoparticles is used and a line that can be seen with the naked eye is shown, so it is widely used for self-diagnosis. Rapid diagnostic tests are currently widely used for the purpose of qualitative analysis to diagnose pregnancy, and to confirm dengue virus and Zika virus infection, and covid-19 antigen or antibody.

Meanwhile, for the purpose of diagnosis, it is required to conduct quantitative analysis for substances such as vitamin D and CRP that measures the level of inflammation in the body, as their production within the body is crucial. However, rapid diagnostic tests currently rely on visual inspection of color intensity on test strips, making quantitative analysis impossible. As a result, they cannot be used for diagnosing substances like vitamin D and CRP.

WO 2019/215199 A1 discloses an analyte testing method for quantifying the presence of an analyte in a specimen by lateral flow chromatography using a mobile hand-held processor device comprising a digital camera for capturing a plurality of images of a test strip having a test line and a control line. §

US 2014/240491 A1 discloses an analyte testing method in a lateral flow assay using a processing arrangement and a digital camera for capturing a plurality of images of a test strip having a test line with a reference line and a control line with a reference line.

### DETAILD DESCRIPTION OF THE INVENTION

### TECHNICAL PROBLEM

The present invention provides a method of performing quantitative analysis in a rapid diagnostic test by means of an existing lateral flow cartridge using marks identifiable by visible light, such as gold nanoparticles.

In addition, the present invention provides a method of easily performing quantitative analysis in point-of-care testing without using separate dedicated equipment. Furthermore, the present invention provides a point-of-care testing method that allows more accurate quantitative analysis by accurately reflecting changes in cartridge characteristics due to manufacturing variations, etc. even after the cartridge is sold.

### TECHNICAL SOLUTION

The present invention to achieve the above-mentioned object provides a method for performing quantitative analysis in a rapid diagnostic test using a lateral flow cartridge containing a mark identifiable by visible light, as defined in independent claim 1.

Preferred embodiments are defined in the dependent claims.

Preferably, the plurality of images are generated from the cartridge video with predetermined time intervals.

Preferably, in the step of identifying a signal measurement area, the identification is performed by recognizing window edges or a specific symbol of the cartridge.

In the case of a different type of mobile communication terminal, the resolution of the camera varies for each device, the way the user takes photos with the camera is different, and the ambient light conditions, etc. are all different, so the cartridge videos obtained by the mobile communication terminals are very different from one another even if the cartridges are the same. Even in this situation, it is necessary to keep the size of the images to be processed by mobile communication terminals (especially, smartphone apps) constant for accurate signal processing. For this purpose, a size guide may be displayed on the display of a mobile communication terminal and an image at that location may be captured, or the distance between the camera and the cartridge may be adjusted using a specific device. In the present invention, even if different images are taken using various types of a mobile communication terminal, the size errors can be minimized by through an algorithm that recognizes not only the size guide but also the edges and specific symbols of the measurement window so as to clearly distinguish the location where the signal is generated and at the same time standardizes the size of the measurement location within the measurement window.

The method according to the invention includes setting a centerline for signal detection in the identified signal measurement area. The signal line generated in a cartridge for a rapid diagnostic test may not be uniform. In this case, qualitative analysis is possible, but errors may occur in quantitative analysis. Accordingly, the method includes the step of generating a control signal by detecting signals above and below the centerline on the control area of the cartridge by means of an analysis algorithm that equalizes the quality of signal lines. The method further includes the step of generating a test signal by detecting signals above and below the centerline in the test area of the cartridge.

Additionally, when the user uses the camera, the focus may be unstable or the unclear or shaky images may be captured, which may result in errors in quantitative analysis. To prevent this, several best images are selected from the images taken through short video recording, and out-of-focus images are discarded, and then two to ten best images are finally chosen, so that optimal signal analysis may be performed using a duplicate review analysis algorithm.

Preferably, in the method for performing quantitative analysis in a rapid diagnostic test, in the step of determining the amount of the analyte, the ratio of the signal intensity is matched to the corresponding formula.

### TECHNICAL EFFECTS

According to the present invention of the above-described configuration, quantitative analysis can be performed in a rapid diagnostic test by means of a lateral flow cartridge containing a mark identifiable by visible light. Additionally, according to the present invention, quantitative analysis can be performed inexpensively and easily without any separate dedicated reading equipment in point-of-care testing. In addition, the present invention provides a point-of-care testing method that enables more accurate quantitative analysis by accurately reflecting the changes in the cartridge characteristics due to manufacturing variations, etc. even after the cartridge is sold. Therefore, the present invention enables tracking and management of cartridges even after product sales. For example, if a claim arises from a customer regarding a product, the cartridge manufacturer can track the same lot and easily provide claim-related information to the users who are performing quantitative analysis.

### BRIEF DESCRIPTION OF THE DRAWINGS

FIG. 1 shows a configuration of a cartridge for rapid diagnostic test to which an embodiment of the present invention is applied.
FIG. 2 illustrates the reaction in a strip of the lateral flow test for quantitative detection of vitamin D metabolites according to an embodiment of the present invention.
FIG. 3 illustrates a process of measuring the reaction results of the cartridge shown in FIG. 1 by means of a mobile communication terminal according to an embodiment of the present invention.
FIG. 4 is a block diagram illustrating the functional blocks and network connection relationship of a mobile communication terminal 300 shown in FIG. 3.
FIG. 5a shows a photograph of the reaction result of the case where the detection pad has a test area and a control area in a lateral flow cartridge equipped with gold nanoparticles as a mark according to an embodiment of the present invention. FIG. 5b illustrates the location of measuring signal strength according to an embodiment of the present invention.
FIG. 6 is a flowchart of a method of performing quantitative analysis on the cartridge shown in Figure 5 according to an embodiment of the present invention.
FIG. 7 is a detailed flowchart of the step of calculating the signal intensity ratio shown in FIG. 6.
FIG. 8 is a photograph of the reaction result of the case where the detection pad of the lateral flow cartridge is provided with a test area, a control area, and an antigen area.
FIG. 9 is a flowchart of a method of performing quantitative analysis on the cartridge shown in FIG. 8 according to another embodiment of the present invention.
FIG. 10 is a detailed flowchart of the step of calculating the signal intensity ratio shown in FIG. 9.
FIG. 11 is a flowchart of a method of writing on a cartridge a quantification formula in accordance with lot change according to an embodiment of the present invention.
FIG 12 is a flowchart of a method for obtaining cartridge characteristic information from a mobile communication terminal according to an embodiment of the present invention.

### BEST MODE FOR ACCOMPLISHING THE INVENTION

Hereinafter, embodiments of the present invention will be described in detail with reference to the accompanying drawings.

In the description below, "lot" is a unit of production, and the product characteristics or process conditions of the same lot are considered to be the same to some extent. Even if products are continuously produced on the same line and under the same conditions, the performance or quality of the products may vary depending on their compartments, their used raw materials, etc. For this reason, actual variations occur even when products are produced under the same conditions, so that management of these variations is required. If all continuous data of actually produced products are measured, the measured values within the same lot vary a little bit, and the values between lots vary even a lot.

"Quantification formula" or "formula" is a function that maps the signal intensity or the signal intensity ratio read by the reading equipment to the content of the analyte.

"Formula file" is the information containing all the contents of the formula, and "formula code" refers to characteristic information of the formula without specific content of the formula.

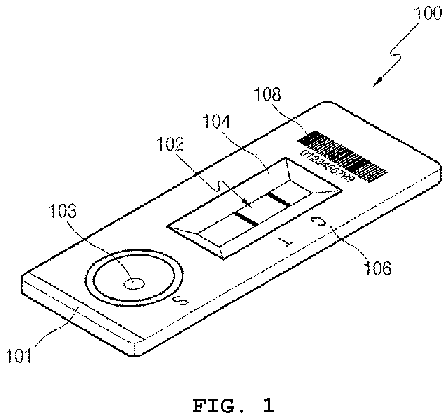

FIG. 1 shows a configuration of a cartridge 100 for rapid diagnostic test to which an embodiment of the present invention is applied. As shown in FIG. 1, cartridge 100 includes a housing 101 and a test strip 102. Test strip 102 is accommodated within housing 101.

The housing 101 has a sample injection inlet 103 and a measurement window 104 on its upper surface. A symbol 106 is written on the upper surface of the housing 101 to indicate the location of a test zone or control zone exposed to the measurement window 104. Additionally, cartridge characteristic information for displaying the analysis object and manufacturing information of the cartridge 100 is written as a QR code 108 on the upper surface of the housing 101.

The test strip 102 is of a lateral flow type and contains a mark identifiable by visible light, such as gold nanoparticles.

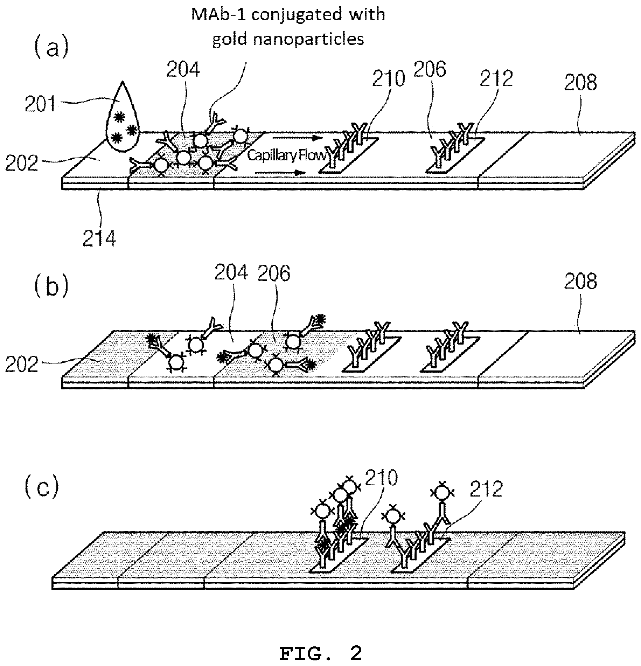

FIG. 2 illustrates the reaction in a strip 102 of the lateral flow test for quantitative detection of vitamin D metabolites according to an embodiment of the present invention. As shown in FIG. 2, the test strip 102 includes a sample pad 202, a conjugation pad 204, a detection pad 206, and an absorbent pad 208. A test area 210 and a control area 212 are formed in the detection pad 206. Support body 214 supports pads 202, 204, 206, and 208.

The sample pad 202 is in fluid communication with the sample injection inlet 103. In some cases, sample pad 202 may include a filtration component configured to leave only the aqueous component by removing the particulate portion of the biological fluid. In addition, sample pad 202 may be selectively treated to modify the capillary flow properties of the material or the properties of the applied sample. For example, the sample pad 202 may be treated with a buffer solution to correct the pH or specific gravity of the applied urine sample or to ensure optimal test conditions.

The biological sample 201 injected into the sample injection inlet 103 is transported to the conjugation pad 204 via the sample pad 202 by capillary flow. The conjugation pad 204 contains a detection reagent. Preferably, the detection reagent is provided as fixed in a dry state and as mobile in a moist state. Detection reagents include specific binding members and marks. "Specific binding members" may be first antibodies that specifically bind to one or more vitamin D molecules. Vitamin D antibodies are well known in the art. An exemplary vitamin D antibody is AF10. "One or more vitamin D molecules" refers to the members of the fat-soluble secosteroids group responsible for enhancing the intestinal absorption of calcium, iron, magnesium, phosphate and zinc. "Labels" can be attached to specific binding members and produce a signal detectable with an optical camera at detection pad 206. The mark may be selected from the group consisting of gold nanoparticles, latex particles, carbon nanoparticles, selenium nanoparticles, and silver nanoparticles. Specific binding members include not only members of immunological pairs such as antigen-antibody, but also members of another specific binding pair, such as ligand-carrier protein, biotin-avidin, hormone-hormone receptor, nucleic acid duplex, IgG-protein A, DNA-DNA, DNA-RNA, etc.

Detection pad 206 may be made of, but not limited to, cellulose (e.g., filter paper, chromatographic paper, nitrocellulose, and materials derived from cellulose acetate) as well as glass fiber, nylon, polyethylene terephthalate, polyvinyl chloride, and polyacrylamide, cross-linked dextran, agarose, polyacrylate, ceramic materials, etc.

The detection pad 206 is an area where discoloration that can be observed with an optical camera appears depending on the reaction results. In this embodiment, the detection pad 206 includes a test area 210 and a control area 212. The detection pad 206 may further include an antigen area, etc., depending on the analysis target.

A second antibody or a detection agent that specifically binds to an immune complex containing a first antibody (eg, AF10) that specifically binds to one or more vitamin D molecules is fixed to the test area 210.

In the control region 212, specific binding members that bind to the first antibody are fixed, no matter whether or not they bind to one or more vitamin D molecules. For example, the specific binding members may be a third antibody capable of binding to the constant region of the first antibody. In some cases, specific binding members may be protein A.

A signal appearing in control area 212 can be used to indicate that test strip 102 is functioning properly. The discoloration of the test area 210 and the control area 212 is detected by an optical camera of a mobile communication terminal.

A biological sample can be any material that is to be tested for the presence or amount of an analyte. Preferably, the biological sample is a fluid sample, more preferably a liquid sample. Examples of liquid samples that can be tested using the cartridge 100 of this embodiment include body fluids such as blood, serum, plasma, saliva, urine, eye secretions, semen, sweat, spinal fluid, etc.

The sample 201 is applied to the sample pad 202 through the sample injection inlet 103. Vitamin D in the sample 201 transported to the conjugation pad 204 specifically binds to the first antibody to create an immune complex. The first antibody can be conjugated with a detection reagent, such as gold nanoparticles. The first antibody that formed an immune complex and the first antibody that did not form an immune complex may flow to the detection pad 206. The detection pad 206 includes a test area 210 and a control area 212. The test area 210 may include a second antibody that specifically binds to a complex of a vitamin D molecule and the first antibody. The first antibody that has formed an immune complex and the first antibody that has not formed an immune complex may flow to the control area 212 and bind to the third antibody so as to be detected in the control area 212. The first antibody that forms an immune complex bound to the second antibody can be detected in the test area 210. The ratio of the level at which the mark attached to the first antibody is detected in the test area 210 to the level (signal intensity) at which the mark is detected in the control area 212 can indicate the level of vitamin D contained in the sample 201. The signal intensity detected in the test area 210 is compared to the signal intensity in the control area 212 to standardize the videos (or images) for variables such as light intensity, light quality, and variations between video devices.

The intensities of the signals detected in the test area 210 and the control area 212 can be read qualitatively with naked eyes, but cannot be measured quantitatively. Therefore, the present invention uses a mobile communication terminal, such as a mobile communication terminal that most users already have, to quantify vitamin D molecules contained in the sample 201.

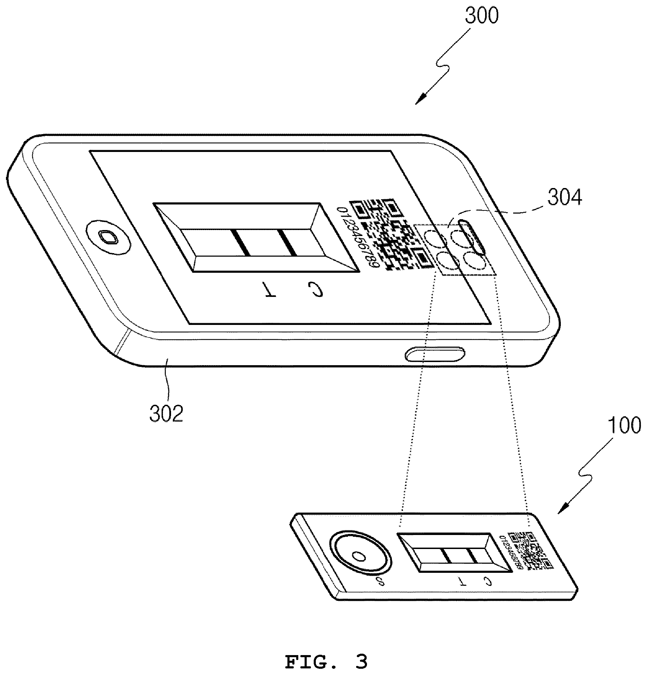

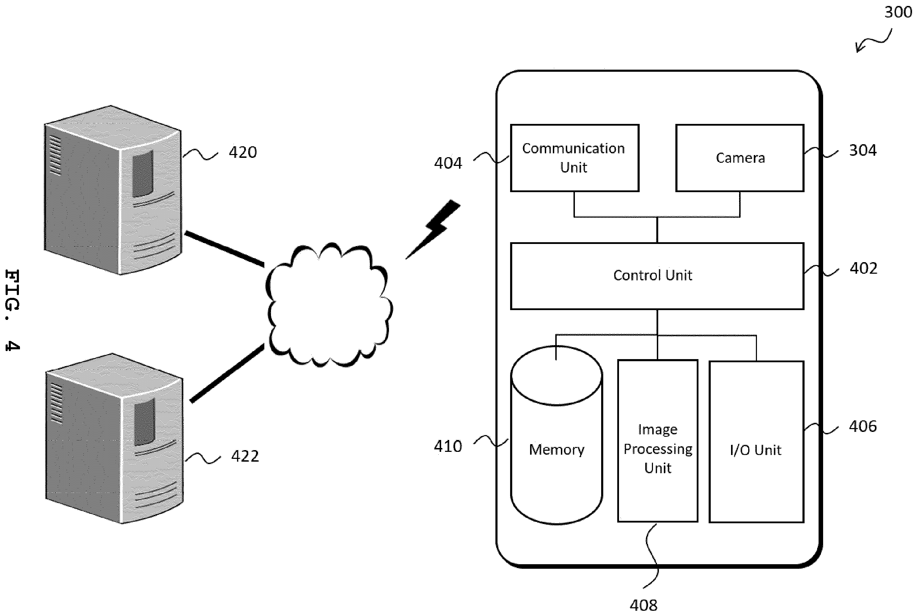

FIG. 3 illustrates a process of measuring the reaction results of the cartridge shown in FIG. 1 by means of a mobile communication terminal 300 according to an embodiment of the present invention. FIG. 4 is a block diagram illustrating the functional blocks and network connection relationship of a mobile communication terminal 300 shown in FIG. 3. A mobile communication terminal 300 is connected to the first service server 420 and the second service server 422 through a network.

As shown in FIGS. 3 and 4, a mobile communication terminal 300 includes a mobile communication terminal body 302 and a camera 304. The main body 302 of a mobile communication terminal includes a control unit 402, a communication unit 404, an input/output unit 406, an image processing unit 408, and a memory 410.

The control unit 402 performs a series of sequences for quantitative analysis according to software stored in the memory 410. The memory 410 receives software configured to quantify one or more vitamin D molecules in a biological sample from the image of the cartridge 100 from a service server (not shown) through a communication unit 404 and stores it. The control unit 402 can receive the quantification formula from the first service server 420 and store it in memory 410. Additionally, the control unit 402 can display the results of quantitative analysis through the input/output unit 406, store them in memory 410, or send them to the second service server 422. The second service server 422 can accumulate and analyze the transmitted analysis results so as to provide the users with various medical services.

The first service server 420 will be managed by the company that manufactures the cartridge 100, but the second service server 422 will be managed by a medical institution that provides medical services. For this reason, the first service server 420 and the second service server 422 are built separately in this embodiment, but they may also be built as one.

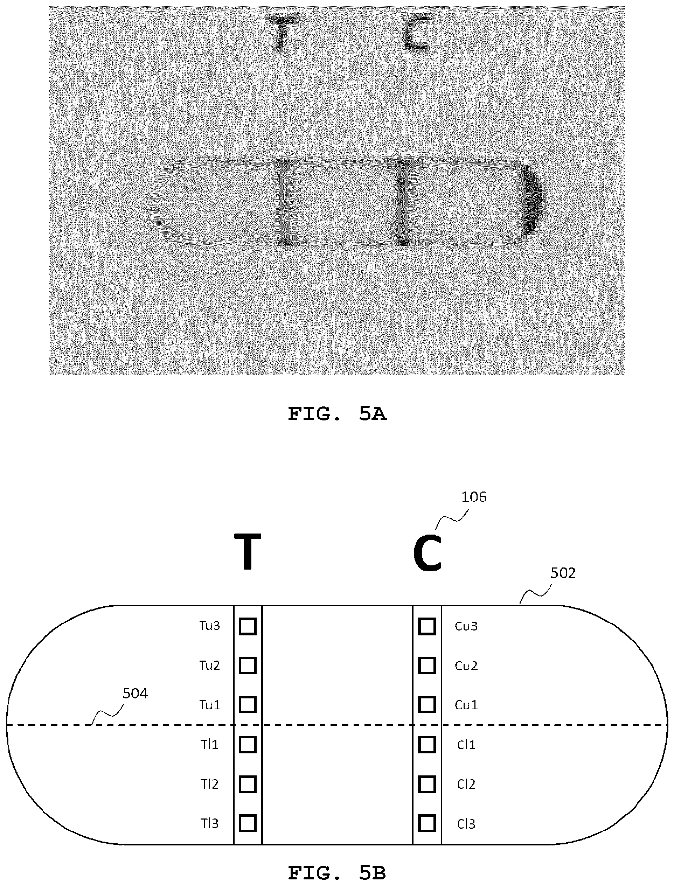

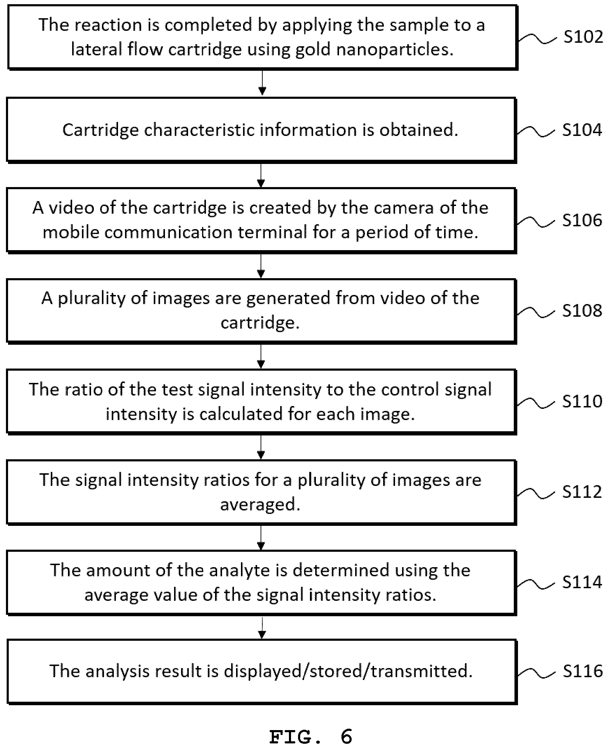

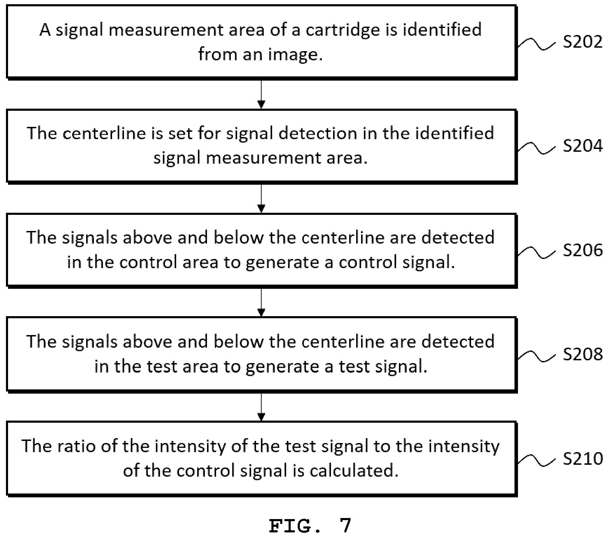

FIG. 5a shows a photograph of the reaction result of the case where the detection pad has a test area (T) and a control area (C) in a lateral flow cartridge equipped with gold nanoparticles as a mark according to an embodiment of the present invention. FIG. 5b illustrates the location of measuring signal strength according to an embodiment of the present invention. FIG. 6 is a flowchart of a method of performing quantitative analysis on the cartridge shown in Figure 5 according to an embodiment of the present invention. The execution order of the steps in FIGS. 6 and 7 may be changed.

First, the reaction is completed by applying the sample to a lateral flow cartridge using gold nanoparticles (S102). Next, the control unit 402 of a mobile communication terminal 300 obtains cartridge characteristic information (S104) and photographs the measurement window 102 with the camera 304 for a predetermined period of time so as to create a video (S106). The control unit 402 obtains information about the cartridge, such as the analysis object, manufacturing information, etc. from the obtained cartridge characteristic information.

The image processing unit 408 generates a plurality of images from the recorded video of the measurement window 102 (S108). The image processing unit 408 may generate images from a video with predetermined time intervals, or may generate a plurality of images when accurate quantitative analysis is possible owing to little movement. Next, the image processing unit 408 calculates the ratio of the test signal intensity to the control signal intensity for each image (S110) and averages the signal intensity ratios for a plurality of images (S112). Next, the control unit 402 determines the amount of the analyte included in the sample using the average value of the signal intensity ratios generated by the image processing unit 408 (S114). To determine the amount of the analyte, the control unit 402 refers to the memory 410 in which a formula for mapping the signal intensity ratio to the amount of the analyte is stored. The control unit 402 displays the determined content of the analyte through the input/output unit 406, stores it in the memory 414, or transmits it to the second service server 422 through a communication unit 404 (S116).

FIG. 7 is a detailed flowchart of the step of calculating the signal intensity ratio shown in FIG. 6.

The image processing unit 408 identifies the signal measurement area from the image generated out of the measurement window video (S202). The signal measurement area can be identified by recognizing the edge 502 or a specific symbol 106 of the measurement window 104. Next, the image processing unit 408 sets the centerline 504 for signal detection in the identified signal measurement area (S204). Next, the image processing unit 408 detects the signals (Cu1, Cu2, Cu3) above the centerline 504 and the signals (Cl1, Cl2, Cl3) below the centerline 504 in the control area (C), and averages them to generate a control signal (S206). Next, the image processing unit 408 detects the signals (Tu1, Tu2, Tu3) above the centerline and the signals (Tl1, Tl2, Tl3) below the centerline in the test area (C), and averages them to generate a test signal (S208). In this embodiment, in order to enable reliable quantitative analysis in the harsh situation of a mobile communication terminal 300 rather than professional reading equipment, i) a plurality of images are generated from a video, and ii) in one image, signals above and below the centerline are detected and averaged to generate reliable control signals and test signals. Next, the image processing unit 408 calculates the ratio of the intensity of the test signal to the intensity of the control signal (S210).

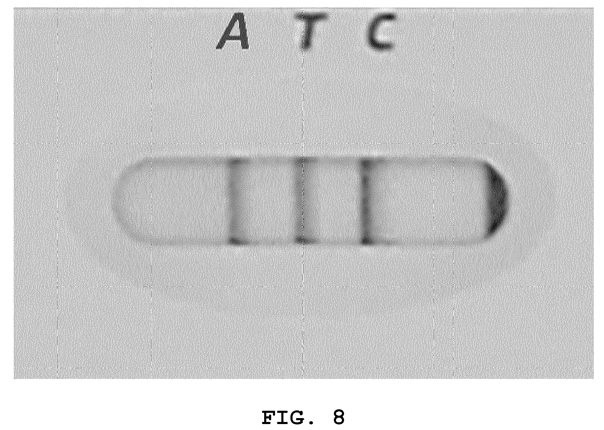

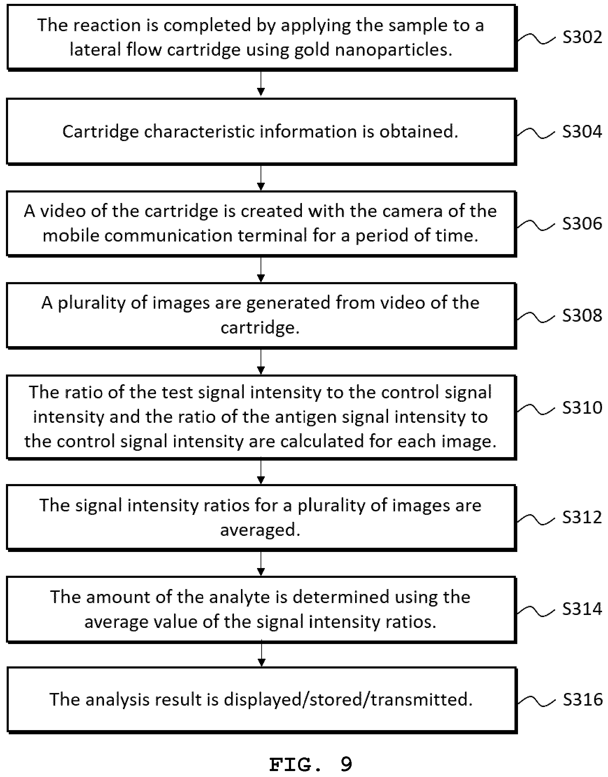

FIG. 8 is a photograph of the reaction result of the case where the detection pad of the lateral flow cartridge is provided with a test area (T), a control area (C), and an antigen area (A). Depending on the analyte, the detection pad of the cartridge further includes a test area (T) and a control area (C) as well as an antigen area (A). FIG. 9 is a flowchart of a method of performing quantitative analysis on the cartridge shown in FIG. 8 according to another embodiment of the present invention. The order of execution of the steps in FIGS. 8 and 9 may be changed.

First, the reaction is completed by applying the sample to a lateral flow cartridge using gold nanoparticles (S302). Next, the control unit 402 of a mobile communication terminal 300 obtains cartridge characteristic information (S304) and films a measurement window 102 by means of the camera 304 for a predetermined period of time (S306). The control unit 402 obtains information about the cartridge, such as analyte and manufacturing information, from the obtained cartridge characteristic information. The image processing unit 408 generates a plurality of images from the captured video of the measurement window 102 (S308). The image processing unit 408 may generate images from a video with predetermined time intervals, or may generate a plurality of images when accurate quantitative analysis is possible owing to little movement. Next, the image processing unit 408 calculates the ratio of the test signal intensity to the control signal intensity and the ratio of the antigen signal intensity to the control signal intensity for each image (S310), and averages the signal intensity ratios (S312). Next, the control unit 402 determines the amount of the analyte contained in the sample using the average value of the signal intensity ratios generated by the image processing unit 408 (S314). To determine the amount of the analyte, the control unit 402 refers to the memory 410 in which a formula for mapping the signal intensity ratio to the amount of the analyte is stored. The control unit 402 displays the determined content of the analyte through the input/output unit 406, stores it in the memory 414, or transmits it to the second service server 422 through a communication unit 404 (S316).

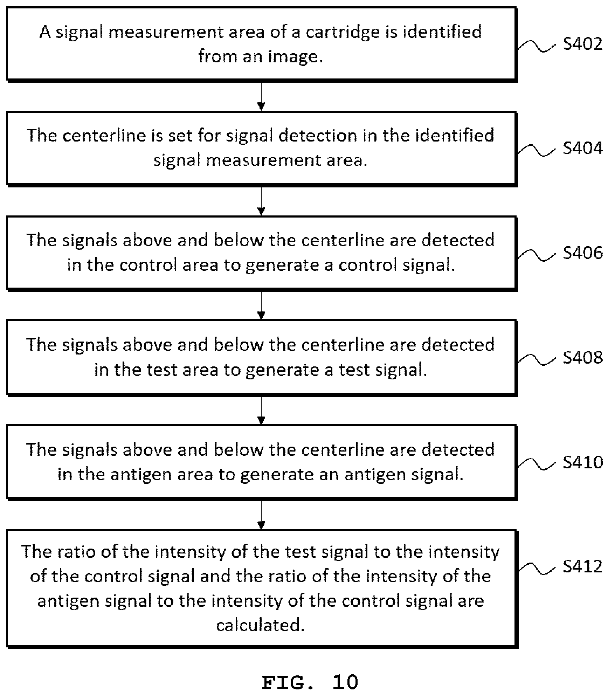

FIG. 10 is a detailed flowchart of the step of calculating the signal intensity ratio shown in FIG. 9.

The image processing unit 408 identifies the signal measurement area from the image generated out of the measurement window video (S402). The signal measurement area can be identified by recognizing the edge or a specific symbol 106 of the measurement window 104. Next, the image processing unit 408 sets the centerline for signal detection in the confirmed signal measurement area (S404). Next, the image processing unit 408 detects the signals above and below the centerline in the control area (C) so to generate a control signal (S406), and detects the signals above and below the centerline in the test area (T) so to generate a test signal (S408). Additionally, the image processing unit 408 generates an antigen signal by detecting signals above and below the centerline in the antigen area A (S410). Next, the image processing unit 408 calculates the ratio of the intensity of the test signal to the intensity of the control signal and the ratio of the intensity of the antigen signal to the intensity of the control signal (S412).

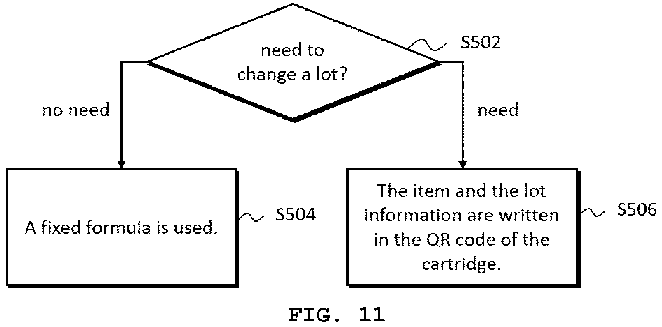

FIG. 11 is a flowchart of a method of writing on a cartridge a quantification formula in accordance with lot change according to an embodiment of the present invention.

First, it is determined whether the cartridge lot needs to be changed (S502). If there is no need to change the lot, the fixed formula is used (S504). In the case that it is necessary to change the formula for mapping signal intensity to content, the cartridge expiration date, etc. due to the changes in the characteristics of the cartridge during the cartridge manufacturing process, item and lot information are written in the QR code 108 of the cartridge 100 (S506).

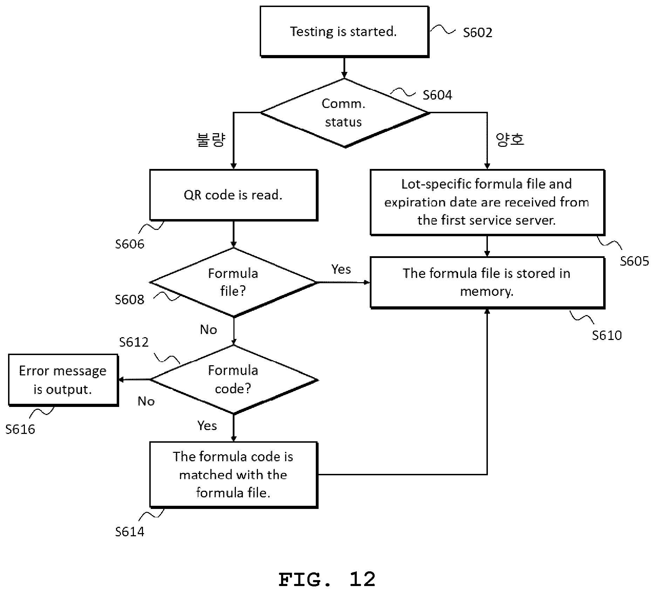

FIG 12 is a flowchart of a method for obtaining cartridge characteristic information from a mobile communication terminal according to an embodiment of the present invention.

When a user selects an item (substance to be measured) on a mobile communication terminal 300 so to start testing (S602), a mobile communication terminal 300 determines the communication status (S604). If it is determined that the communication status is good, the lot-specific formula file and expiration date are received from the first service server 420 (S605) and stored in the memory 410 of a mobile communication terminal 300 (S610).

If the communication condition is poor, a mobile communication terminal 300 reads the QR code 108 of the cartridge 100 using the camera 304 (S606). It is determined whether a formula file is written in the read QR code (S608), and if there is a formula file, it is stored in memory 410 (S610). If there is no formula file, it is determined whether the read QR code has a formula code (S612). If there is a formula code, the formula code is matched with the formula file stored in the memory 410, etc. to select a formula for quantification (S614). If there is no formula code, an error message is output or quantification is performed using the default formula (S616).

By using the method described in FIGS. 11 and 12, this embodiment enables more accurate quantitative analysis by obtaining in almost real time a formula that accurately reflects variation in cartridge characteristics, such as manufacturing variation. Additionally, this embodiment enables tracking and management of cartridges even after product sales. For example, if a customer complains about a product, the cartridge manufacturer can track the same lot and easily provide complaint-related information to the customer who is performing quantitative analysis.

As described above, although the embodiments have been described with limited examples and drawings, those skilled in the art will understand that various modifications and variations are possible within the scope of protection of the present invention which is defined by the appended claims.

## Claims

1. A method for performing quantitative analysis in a rapid diagnostic test using a lateral flow cartridge containing a mark identifiable by visible light, including steps of:
(a) preparing a cartridge (100) where a sample is applied and reaction is completed (S102);
(b) generating cartridge video by photographing a mark identifiable by visible light by means of a camera (304) of a mobile communication terminal (300) with predetermined time intervals (S106), the mark generated as a result of the reaction of the cartridge;
(c) generating a plurality of images from the cartridge video (S108);
(d) identifying a signal measurement area of the cartridge from an image (S202, S402);
(e) setting a centerline (504) for signal detection in the identified signal measurement area (S204, S404), and
(f) generating a control signal (S206, S406) by averaging signals (Cu1, Cu2, Cu3) above and signals (Cl1, Cl2, Cl3) below the centerline on the control area (C) of the cartridge, and generating a test signal (S208, S408) by averaging signals (Tu1, Tu2, Tu3) above and signals (Tl1, Tl2, Tl3) below the centerline in the test area of the cartridge,
(g) calculating the ratio of the test signal intensity to the control signal intensity for each of the plurality of images (S210);
(h) averaging signal intensity ratios for the plurality of images (S112); and
(i) determining the amount of the analyte contained in the sample using the averaged signal intensity ratio (S114, S314).

2. The method for performing quantitative analysis in a rapid diagnostic test of claim 1, wherein the plurality of the images are generated from the cartridge video with predetermined time intervals.

3. The method for performing quantitative analysis in a rapid diagnostic test of claim 1, wherein in the step (d) of identifying a signal measurement area, the identification is performed by recognizing window edges (502) or a specific symbol (106) of the cartridge.

4. The method for performing quantitative analysis in a rapid diagnostic test of claim 1, wherein in the step (i) of determining the amount of the analyte, the ratio of the signal intensity is matched to the corresponding formula.

## Patentansprüche

1. Verfahren zum Durchführen quantitativer Analyse in einem diagnostischen Schnelltest unter Verwendung einer Lateral-Flow-Kartusche, die eine mit sichtbarem Licht identifizierbare Markierung enthält, umfassend folgender Schritte:
(a) Vorbereiten einer Kartusche (100), auf die eine Probe aufgebracht wird und eine Reaktion abgeschlossen wird (S102);
(b) Erzeugen von Kartuschenvideo durch Fotografieren einer Markierung, die mit sichtbarem Licht mittels einer Kamera (304) eines Mobilfunkendgeräts (300) mit vorgegebenen Zeitintervallen (S106) identifizierbar ist, wobei die Markierung als ein Ergebnis der Reaktion der Kartusche erzeugt wird;
(c) Erzeugen von mehreren Bildern aus dem Kartuschenvideo (S108);
(d) Identifizieren eines Signalmessbereichs der Kartusche aus einem Bild (S202, S402);
(e) Einstellen einer Mittellinie (504) für Signalerfassung in dem identifizierten Signalmessbereich (S204, S404), und
(f) Erzeugen eines Steuersignals (S206, S406) durch Mittelwertbildung von Signalen (Cu1, Cu2, Cu3) oberhalb und Signalen (Cl1, Cl2, Cl3) unterhalb der Mittellinie in dem Steuerbereich (C) der Kartusche, sowie Erzeugen eines Testsignals (S208, S408) durch Mittelwertbildung von Signalen (Tu1, Tu2, Tu3) oberhalb und Signalen (Tl1, Tl2, Tl3) unterhalb der Mittellinie in dem Testbereich der Kartusche,
(g) Berechnen des Verhältnisses der Testsignalintensität zu der Kontrollsignalintensität für jedes der mehreren Bilder (S210);
(h) Mittelwertbilden der Signalintensitätsverhältnisse für die mehreren Bilder (S112); und
(i) Bestimmen der Menge des Analyten, der in der Probe enthalten ist, unter Verwendung des gebildeten Mittelwertes des Signalintensitätsverhältnisses (S114, S314).

2. Verfahren zum Durchführen quantitativer Analyse in einem diagnostischen Schnelltest nach Anspruch 1, wobei die mehreren Bilder aus dem Kartuschenvideo mit vorbestimmten Zeitintervallen erzeugt werden.

3. Verfahren zum Durchführen quantitativer Analyse in einem diagnostischen Schnelltest nach Anspruch 1, wobei in dem Schritt (d) von Identifizieren eines Signalmessbereichs die Identifizierung durch Erkennen von Fensterkanten (502) oder eines spezifischen Symbols (106) der Kartusche durchgeführt wird.

4. Verfahren zum Durchführen quantitativer Analyse in einem diagnostischen Schnelltest nach Anspruch 1, wobei in dem Schritt (i) von Bestimmen der Menge des Analyten das Verhältnis der Signalintensität mit der entsprechenden Formel abgeglichen wird.

## Revendications

1. Une méthode pour effectuer une analyse quantitative dans un test de diagnostic rapide à l'aide d'une cartouche à flux latéral contenant une marque identifiable par lumière visible, comprenant les étapes comprenant le fait de :
(a) préparer une cartouche (100) sur laquelle un échantillon est appliqué et une réaction est achevée (S102) ;
(b) générer une vidéo de la cartouche en photographiant une marque identifiable par lumière visible au moyen d'une caméra (304) d'un terminal de communication mobile (300) à des intervalles de temps prédéterminés (S106), la marque étant générée à la suite de la réaction de la cartouche ;
(c) générer une pluralité d'images à partir de la vidéo de la cartouche (S108) ;
(d) identifier une zone de mesure de signal de la cartouche à partir d'une image (S202, S402) ;
(e) définir une ligne centrale (504) en vue d'une détection de signal dans la zone de mesure de signal identifiée (S204, S404), et
(f) générer un signal de contrôle (S206, S406) par moyennage des signaux (Cu1, Cu2, Cu3) situés au-dessus et des signaux (Cl1, Cl2, Cl3) situés au-dessous de la ligne centrale sur la zone de contrôle (C) de la cartouche, et générer un signal de test (S208, S408) par moyennage des signaux (Tu1, Tu2, Tu3) situés au-dessus et des signaux (TI1, Tl2, Tl3) situés au-dessous de la ligne centrale dans la zone de test de la cartouche,
(g) calculer le rapport de l'intensité du signal de test à l'intensité du signal de contrôle pour chacune de la pluralité d'images (S210) ;
(h) moyenner les rapports d'intensité du signal pour la pluralité d'images (S112) ; et
(i) déterminer la quantité de l'analyte contenu dans l'échantillon à l'aide du rapport d'intensité du signal moyenné (S114, S314).

2. La méthode pour effectuer une analyse quantitative dans un test de diagnostic rapide selon la revendication 1, dans lequel la pluralité d'images est générée à partir de la vidéo de la cartouche à des intervalles de temps prédéterminés.

3. La méthode pour effectuer une analyse quantitative dans un test de diagnostic rapide selon la revendication 1, dans lequel, à l'étape (d) d'identification d'une zone de mesure de signal, l'identification est effectuée par reconnaissance de bords de fenêtre (502) ou d'un symbole spécifique (106) de la cartouche.

4. La méthode pour effectuer une analyse quantitative dans un test de diagnostic rapide selon la revendication 1, dans lequel, à l'étape (i) de détermination de la quantité de l'analyte, le rapport de l'intensité du signal est mis en correspondance avec la formule correspondante.
